# EUROPÄISCHE PATENTSCHRIFT

(11) **EP 3 283 001 B1**
(45) Veröffentlichungstag und Bekanntmachung des Hinweises auf die Patenterteilung: **20.11.2019**
(21) Anmeldenummer: 16723024.2
(22) Anmeldetag: 18.04.2016
(51) Int. Cl.: B23B 31/20, A61C 1/14, B23B 31/117, A61B 17/16

(54) **SPANNVORRICHTUNG ZUM EINSPANNEN EINES ZYLINDERFÖRMIGEN WERKZEUGSCHAFTS**
CLAMPING DEVICE FOR CLAMPING A CYLINDRICAL INSTRUMENT SHANK
DISPOSITIF DE SERRAGE D'UNE TIGE D'OUTIL CYLINDRIQUE

(30) Priorität: 16.04.2015 DE 102015206904
(43) Veröffentlichungstag der Anmeldung: 21.02.2018
(73) Patentinhaber: Sirona Dental Systems GmbH, 64625 Bensheim (DE)
(72) Erfinder: ERTUGRUL, Metin, 64295 Darmstadt (DE); GOISSER, Siegfried, 64683 Einhausen (DE); GÖBEL, Stefan, 63225 Langen (DE)
(74) Vertreter: Özer, Alpdeniz
(86) Internationale Anmeldenummer: PCT/EP2016/058523
(87) Internationale Veröffentlichungsnummer: WO 2016/166367

(56) Entgegenhaltungen:
- EP-A1- 1 709 930
- EP-A1- 2 196 274
- US-B2- 7 303 394

## Beschreibung

### Technisches Gebiet

Die Erfindung betrifft eine Spannvorrichtung zum Einspannen eines zylinderförmigen Werkzeugschafts, insbesondere eines zahnärztlichen Werkzeugs, aufweisend ein Federelement zum Halten des Werkzeugschafts, eine hohle Welle zum Übertragen einer Drehbewegung, und einen Stößel, wobei das Federelement als Hülse mit einer zylinderförmigen Wand, einer ersten Öffnung und einer zweiten Öffnung ausgebildet ist und die Wand Ausnehmungen aufweist.

### Stand der Technik

Aus dem Stand der Technik sind verschiedene Varianten von Spannvorrichtungen zum Einspannen eines in Drehung zu versetzenden zylindrischen Werkzeugschafts bekannt.

Die in der EP 0 273 259 A1 beschriebene Spannvorrichtung umfasst eine Spannhülse mit durch Längsschlitze gebildeten Zungen, die den Werkzeugschaft halten. Kontakt- bzw. Halteflächen bilden sich insbesondere an den Kanten aus.

Daher ist es auch bekannt bzw. üblich, diese besonders belasteten Bereiche aus einem korrosionsbeständigen, harten Material auszubilden oder mit einem solchen zu beschichten.

Aus der DE 33 46 248 A1 ist beispielsweise eine Spanneinrichtung zum Einspannen eines zahnärztlichen Werkzeugs mit einer zweiteiligen Klemmvorrichtung bekannt. Die Klemmkraft wird durch ein Federelement erbracht und durch baulich getrennte Klemmbacken auf den zu haltenden Werkzeugschaft übertragen. Dies ermöglicht es einerseits, die in Kontakt mit dem Werkzeugschaft stehenden Flächen aus einem möglichst harten, korrosionsbeständigen Material herzustellen und erleichtert andererseits die Wartung, da bei auftretendem Verschleiß der Kontaktflächen lediglich die Klemmbacken getauscht werden müssen.

Ferner wird noch die US 7 303 394 B2 genannt.

Nachteilig an den vorbeschriebenen Spannvorrichtungen sind die verhältnismäßig geringen Ausmaße der haltenden Flächen bzw. Kanten und die hierdurch sehr hohen Krafteinträge. Aufgabe der Erfindung ist es daher, eine besonders belastbare Spannvorrichtung bereitzustellen.

Diese Aufgabe wird durch eine Spannvorrichtung zum Einspannen eines zylinderförmigen Werkzeugschafts, insbesondere eines zahnärztlichen Werkzeugs, gemäß Anspruch 1 gelöst.

Das beispielsweise über eine Außenwelle auf die Welle übertragene Drehmoment kann direkt und/oder über das Federelement und/oder den Stößel auf einen im Federelement gehaltenen Werkzeugschaft übertragen werden. Der Werkzeugschaft wird im Federelement kraftschlüssig und/oder formschlüssig gehalten. Dabei wird ein Kraftschluss durch eine radiale Federkraft des Federelements umgesetzt. Ein Formschluss kann beispielsweise mittels einer Nut am Federelement und einem entsprechend ausgeformten Vorsprung, z.B. Stift oder Zapfen, am Werkzeugschaft oder in anderer bekannter Weise umgesetzt werden.

Das erfindungsgemäße Federelement stellt ohne weitere Zwischenteile den Kontaktpartner zum Werkzeug dar, wodurch eine kleine und kompakte Bauweise der Spannvorrichtung ermöglicht wird.

Durch das beidseitige Eingreifen eines Bauteils, nämlich von Welle und Stößel, in das Federelement, wird eine Betätigungskraft zum Öffnen bzw. Weiten des Federelements doppelt genutzt und kann entsprechend geringer ausfallen.

Die hülsenförmige Ausgestaltung des Federelements stellt ferner einen möglichst langen elastisch federnden Bereich und eine radial wirkende Kontaktkraft bereit. Hierdurch wird ein geringer Verschleiß und eine gute Rundlaufgenauigkeit erreicht. Da der Vorspannweg in Klemmstellung signifikant größer als die Toleranzschwankungen der geklemmten Werkzeugschaftdurchmesser ist, ergeben sich ferner nur kleine Schwankungen der Haltekräfte und eine große Toleranz gegen Abweichungen des Werkzeugschaftdurchmessers.

Die Ausnehmungen in der Wand des Federelements ermöglichen eine optimale Werkstoffausnutzung, um die maximale Beanspruchung möglichst gering zu halten und so eine große Anzahl möglicher Werkzeugwechsel sicherzustellen. Hierfür kann insbesondere die spezielle Gestaltung hinsichtlich Breite, Länge und Anzahl der Ausnehmungen optimiert werden. Beispielsweise kann durch beidseitig versetzte Schlitze eine schleifenartige Form erreicht werden, die längliche elastische Halteflächen ermöglicht. Je breiter solche Schlitze ausgebildet werden, um so mehr degeneriern die Halteflächen allerdings zu Linien.

Das Federelement kann auch kreisförmige Ausnehmungen, z.B. Bohrungen, aufweisen, die beispielsweise gleichmäßig über den gesamten Mantel des Federelements verteilt sind. Eine weitere Variante ist ein durch Rautenmaschen geschwächter Grundkörper, also ein Federelement mit einem Mantel mit in Reihen und Spalten angeordneten rautenförmigen Ausnehmungen.

Die geringe Anzahl von Bauteilen der Spannvorrichtung und die Einfachheit dieser Bauteile ermöglichen eine besonders kostengünstige Fertigung.

Vorteilhafterweise ist das Federelement formschlüssig und/oder kraftschlüssig mit der Welle verbunden. Die Drehbewegung kann hierdurch direkt von der Welle auf das Federelement übertragen werden. Ein Formschluss kann beispielsweise durch einen Vorsprung an der Welle und eine entsprechende Ausnehmung in der Wand des Federelements oder durch einen Vorsprung, z.B. einen Zapfen, am Federelement und eine entsprechende Ausnehmmung, z.B. Nut, an der Welle erreicht werden.

Vorteilhafterweise sind die Ausnehmungen in der Wand des Federelements als zumindest näherungsweise parallel zur Drehachse des Federelements verlaufende Schlitze ausgebildet, wobei mindestens ein Schlitz an der ersten Öffnung des Federelements beginnt und mindestens ein zweiter Schlitz an der zweiten Öffnung beginnt, um eine möglichst über die gesamte Höhe des Federelements wirkende radiale Federkraft bereitzustellen. Vorteilhaft für eine einfache Fertigung ist eine gerade Anzahl von Schlitzen, beispielsweise vier oder sechs Schlitze.

Vorteilhafterweise sind die an der ersten Öffnung beginnenden Schlitze in einer Umfangsrichtung des Federlements versetzt zu den an der zweiten Öffnung beginnenden Schlitzen angeordnet. Hierdurch wird eine schleifenartige Form des Federelements erreicht, die auch in kleinem Bauraum einen langen, elastisch federnden Bereich ermöglicht.

Vorteilhafterweise weisen die Schlitze an einem von der Öffnung des Federelements abgewandten Ende eine weitere Ausnehmung auf, wodurch die Elastizität des Federelements erhöht wird.

Vorteilhafterweise besteht das Federelement aus einem säure- und korrosionsbeständigen Material. Hierdurch kann die Haltbarkeit des Federelements erhöht werden, welches als direkter Kontaktpartner zum Werkzeug besonders anfällig für Verschleiß ist. Natürlich ist zur Erhöhung der Haltbarkeit statt einer vollständigen Ausformung des Federelements aus einem säure- und korrosionsbeständigem Material auch eine Beschichtung der Innenflächen, also der besonders vom Verschleiß betroffenen Kontaktflächen mit dem Werkzeug, mit einem solchen Material möglich.

Vorteilhafterweise weist das Federelement um die erste und die zweite Öffnung herum, nach innen schräg abfallende Betätigungsflächen auf, um ein einfaches Hineinbewegen von Stößel und Welle in das Federelement zu ermöglichen.

Ferner betrifft die Erfindung ein dentales Präparationsinstrument, welches eine Spannvorrichtung der vorbeschriebenen Art umfasst.

### Kurzbeschreibung der Zeichnungen

Ausführungsbeispiele der Erfindung sind in den Zeichnungen dargestellt. Es zeigt die
- Fig. 1: eine schematische Darstellung einer erfindungsgemäßen Spannvorrichtung, die
- Fig. 2A, B: eine erste und eine zweite Ausführungsform eines erfindungsgemäßen Federelements, die
- Fig. 3A, B: eine erste und eine zweite Ausführungsform einer Welle für eine erfindungsgemäße Spannvorrichtung, die
- Fig.4: eine schematische Darstellung einer weiteren Ausführungsform der Spannvorrichtung und die
- Fig.5: verschiedene Ausführungsformen der Ausnehmungen der Wand des Federelements.

### Ausführungsbeispiele

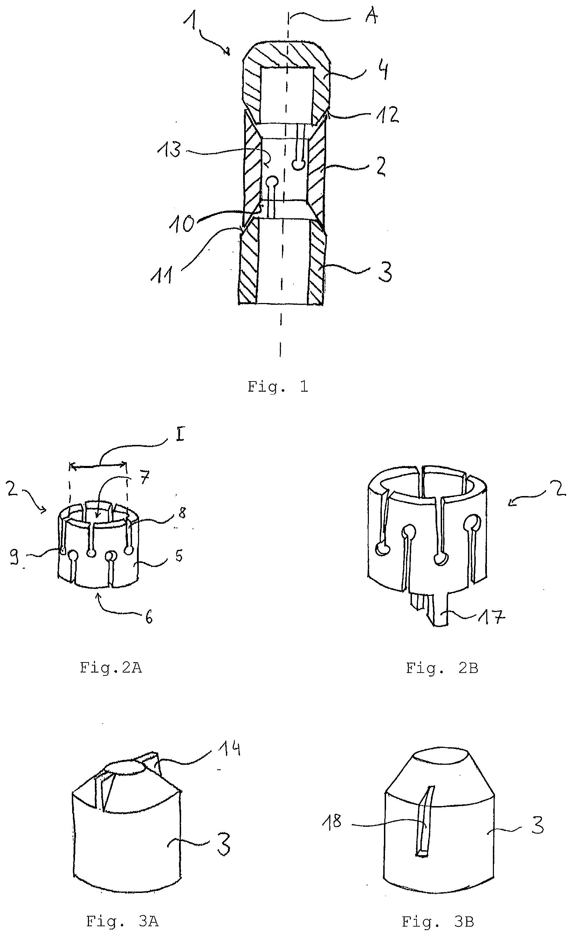

Die Fig. 1 zeigt eine erfindungsgemäße Spannvorrichtung 1, welche ein Federelement 2, eine Welle 3 und einen Stößel 4 aufweist. Federelement 2, Welle 3 und Stößel 4 weisen jeweils eine Rotationsachse auf und sind hinsichtlich dieser Achse A koaxial angeordnet.

Das Federelement 2, welches einzeln in Fig. 2A skizziert ist, ist als Hülse mit einer zylinderförmigen Wand 5, einer ersten Öffnung 6 und einer zweiten Öffnung 7 ausgebildet und weist einen Innendurchmesser I auf, der etwas kleiner ist, als ein Durchmesser eines einzuspannenden Werkzeugschafts.

In der in Fig. 2A gezeigten Ausführungsform wird eine radiale Weitung des Federelements 2 bzw. eine radial wirkende Federkraft durch beidseitig, versetzt angeordnete schlitzförmige Ausnehmungen 8, die sich von den beiden Öffnungen 6, 7 des hülsenförmigen Federelements 2 jeweils in Richtung der gegenüberliegenden Öffnung 6, 7 erstrecken, möglich. Die schlitzförmigen Ausnehmungen 8 enden in dem dargestellten Ausführungsbeispiel in weiteren kreisförmigen Ausnehmungen 9.

Aus fertigungstechnischen Gründen ist eine gerade Anzahl von Schlitzen je Seite zu bevorzugen, wie beispielsweise in Fig. 2B dargestellt.

Ferner weist das Federelement 2 gemäß der in Fig. 1 dargestellten Ausführungsform schräg nach innen abfallende Betätigungsflächen 10 auf. Die Welle 3 und der Stößel 4 weisen entsprechende schräg nach außen abfallende Betätigungsfläche 11, 12 auf, so dass das teilweise Einführen von Welle 3 und Stößel 4 in jeweils ein Ende des Federelements 2 erleichtert wird. Die schräg abfallenden Betätigungsflächen 10, 11, 12 können beispielsweise konusförmig oder pyramidenstumpfförmig ausgebildet sein.

Durch das Hineinbewegen von Welle 3 und/oder Stößel 4 in das Federelement 2, wird dieses durch eine axial aufgebrachte Betätigungskraft geweitet bzw. geöffnet. Die Bewegung der als Kontaktflächen mit dem Werkzeugschaft bzw. Halteflächen für den Werkzeugschaft dienende Innenfläche 13 des Federelements 2 erfolgt dabei aufgrund der erfindungsgemäßen koaxialen Anordnung der hülsenförmigen und ineinander greifenden Bauteile rein radial. Die Innenfläche 13 bleibt weitestgehend parallel zur Rotationsachse A der Spannvorrichtung 1. Hierdurch wird ein gleichmäßiges Anlegen der als Kontaktflächen bzw. Halteflächen dienenden Innenfläche 13 auch für unterschiedliche Werkzeugschaftdurchmesser erreicht.

Werden Welle 3 und Stößel 4 wieder aus dem Federelement 2 herausbewegt, zieht sich dieses in radialer Richtung zusammen und hält einen eingeführten Werkzeugschaft an der gesamten Innenfläche 13. Hierdurch wird eine möglichst geringe Flächenpressung erreicht und so der Verschleiß verringert.

Um eine Übertragung der Drehbewegung von der Welle 3 auf ein Werkzeug zu ermöglichen, kann die Welle 3, wie in Fig. 3A skizziert, einen Vorsprung 14 für eine formschlüssige Verbindung mit dem das Werkzeug haltenden Federelement 2 bzw. einer entsprechenden Ausnehmung des Federelements 2 aufweisen.

Ebenfalls möglich ist ein Formschluss zwischen Federlement 2 und Welle 3 durch einen oder mehrere am Federelement 2 angeordnete Vorsprünge 17 und eine oder mehrere entsprechende Ausnehmungen 18 an der Welle 3, wie in den Figuren 2B und 3B dargestellt.

Auch eine rein kraftschlüssige Verbindung zwischen Welle und Federelement oder eine kraft- und/oder formschlüssige Verbindung zwischen dem Federelement und einem weiteren die Drehbewegung übertragenden Bauteil ist möglich.

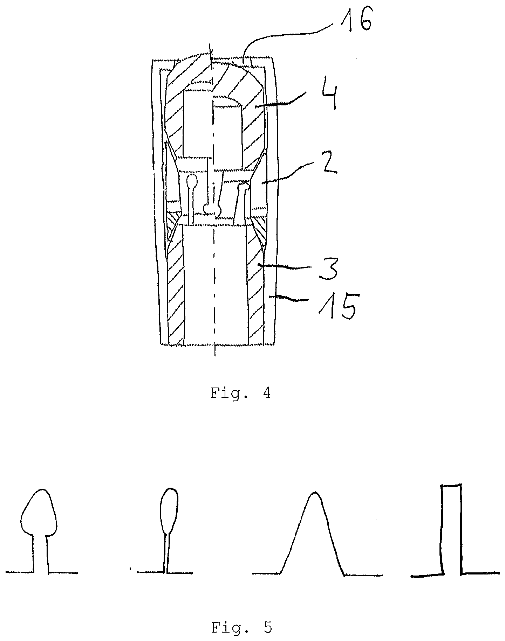

Die Spannvorrichtung 1 kann auch, wie in der Ausführungsform in Fig. 4 skizziert, zusätzlich eine Außenwelle 15 aufweisen, die ein Drehmoment auf die Welle 3 oder auf Welle 3 und Federelement 2 überträgt, wozu die Bauteile kraft- und/oder formschlüssig miteinander verbunden sind. Gemäß der Ausführungsform der Fig. 4 umschließt die Außenwelle 15 die Welle 3, den Stößel 4 sowie das Federelement 2 und weist eine Öffnung 16 und/oder ein Betätigungselement zur Betätigung des Stößels 4 in einer bekannten Weise auf.

Die Ausnehmungen 8 zur Schwächung der Wand 5 des Federelements 2 können unterschiedlichste Formen haben. Beispiele sind in Fig. 5 dargestellt.

### Bezugszeichenliste

- 1: Spannvorrichtung
- 2: Federelement
- 3: Welle
- 4: Stößel
- 5: Wand des Federelements
- 6: erste Öffnung des Federelements
- 7: zweite Öffnung des Federelements
- 8: Ausnehmungen
- 9: Ausnehmungen
- 10: Betätigungsfläche des Federelements
- 11: Betätigungsfläche der Welle
- 12: Betätigungsfläche des Stößels
- 13: Innenfläche des Federelements
- 14: Vorsprung
- 15: Außenwelle
- 16: Öffnung
- 17: Vorsprung
- 18: Ausnehmung
- A: Rotationsachse der Spannvorrichtung
- I: Innendurchmesser

## Patentansprüche

1. Spannvorrichtung (1) zum Einspannen eines zylinderförmigen Werkzeugschafts, insbesondere eines zahnärztlichen Werkzeugs, aufweisend ein Federelement (2) zum Halten des Werkzeugschafts, eine hohle Welle (3) zum Übertragen einer Drehbewegung, und einen Stößel (4), wobei das Federelement (2) als Hülse mit einer zylinderförmigen Wand (5), einer ersten Öffnung (6) und einer zweiten Öffnung (7) ausgebildet ist und die Wand Ausnehmungen (8) aufweist, wobei das Federelement (2), die Welle (3) und der Stößel (4) Drehachsen aufweisen, die koaxial angeordnet sind, die Welle (3) zum Weiten des Federelements (6) in die erste Öffnung (6) des Federelements (2) zumindest teilweise hineinragt und der Stößel (4) zum Weiten des Federelements (6) in die zweite Öffnung (7) des Federelements (2) zumindest teilweise hineinragt und das Federelement (2) formschlüssig und/oder kraftschlüssig mit der Welle (3) verbunden ist.

2. Spannvorrichtung (1) nach Anspruch 1, **dadurch gekennzeichnet, dass** die Ausnehmungen (8) in der Wand des Federelements (2) als zumindest näherungsweise parallel zur Drehachse des Federelements (2) verlaufende Schlitze ausgebildet sind, wobei mindestens ein Schlitz an der ersten Öffnung (6) des Federelements (2) beginnt und mindestens ein zweiter Schlitz an der zweiten Öffnung (7) beginnt.

3. Spannvorrichtung (1) nach Anspruch 2, **dadurch gekennzeichnet, dass** die an der ersten Öffnung (6) beginnenden Schlitze in Umfangsrichtung des Federelements (2) versetzt zu den an der zweiten Öffnung (7) beginnenden Schlitzen angeordnet sind.

4. Spannvorrichtung (1) nach Anspruch 2 oder 3, **dadurch gekennzeichnet, dass** die Schlitze an einem von der Öffnung (6, 7) des Federelements (2) abgewandten Ende eine weitere Ausnehmung (9) aufweisen.

5. Spannvorrichtung (1) nach einem der Ansprüche 1 bis 4, **dadurch gekennzeichnet, dass** das Federelement (2) aus einem säure- und korrosionsbeständigen Material besteht.

6. Spannvorrichtung (1) nach einem der Ansprüche 1 bis 5, dass das Federelement (2) um die erste und die zweite Öffnung (6, 7) herum, nach innen schräg abfallende Betätigungsflächen (10) aufweist.

7. Zahnmedizinisches Präparationsinstrument, **dadurch gekennzeichnet, dass** das Präparationsinstrument eine Spannvorrichtung (1) nach einem der vorangehenden Ansprüche umfasst.

## Claims

1. Clamping device (1) for clamping a cylindrical tool shaft, in particular of a dental tool, comprising a spring element (2) for holding the tool shaft, a hollow shaft (3) for transmitting a rotational movement, and a plunger (4), wherein
the spring element (2) is configured as a sleeve having a cylindrical wall (5), a first opening (6) and a second opening (7) and the wall comprises recesses (8), wherein the spring element (2), the shaft (3) and the plunger (4) have coaxially disposed rotational axes, the shaft (3) projects at least partially into the first opening (6) of the spring element (2) in order to expand the spring element (6) and the plunger (4) projects at least partially into the second opening (7) of the springloaded element (2) in order to expand the spring element (6), and the spring element (2) is connected to the shaft (3) in a form-fitting or force-fitting manner.

2. Clamping device (1) according to Claim 1, **characterized in that** the recesses (8) in the wall of the spring element (2) are configured as slots which extend at least approximately parallel to the rotational axis of the spring element (2), wherein at least one slot starts at the first opening (6) of the spring element (2) and at least one second slot starts at the second opening (7).

3. Clamping device (1) according to Claim 2, **characterized in that** the slots which start at the first opening (6) are arranged offset in the circumferential direction of the spring element (2) to the slots which start at the second opening (7).

4. Clamping device (1) according to Claim 2 or 3, **characterized in that** the slots comprise a further recess (9) on an end which faces away from the opening (6, 7) of the spring element (2).

5. Clamping device (1) according to any one of Claims 1 to 4, **characterized in that** the spring element (2) consists of an acid and corrosion-resistant material.

6. Clamping device (1) according to any one of Claims 1 to 5, [*sic*] that the spring element (2) comprises actuating surfaces (10) around the first and the second opening (6, 7) which slope inward at an angle.

7. Dental preparation instrument, **characterized in that** the preparation instrument comprises a clamping device (1) according to any one of the preceding claims.

## Revendications

1. Dispositif de serrage (1) pour le serrage d'une tige d'outil cylindrique, en particulier d'une tige d'outil dentaire, comprenant un élément ressort (2) pour le maintien de la tige d'outil, un arbre creux (3) pour la transmission d'un mouvement de rotation et un coulisseau (4), l'élément ressort (2) étant conçu comme une gaine comprenant une paroi cylindrique (5), une première ouverture (6) et une deuxième ouverture (7) et la paroi présentant des cavités (8),
l'élément ressort (2), l'arbre (3) et le coulisseau (4) présentant des axes de rotation disposés de manière coaxiale, l'arbre (3) s'étendant au moins partiellement dans la première ouverture (6) de l'élément ressort (2) pour l'élargissement de l'élément ressort (6) et le coulisseau (4) s'étendant au moins partiellement dans la deuxième ouverture (7) de l'élément ressort (2) pour l'élargissement de l'élément ressort (6) et l'élément ressort (2) étant connecté en solidarité de forme ou de force à l'arbre (3).

2. Dispositif de serrage (1) selon la revendication 1, **caractérisé en ce que** les cavités (8) dans la paroi de l'élément ressort (2) sont réalisées sous la forme d'encoches s'étendant au moins approximativement parallèlement à l'axe de rotation de l'élément ressort (2), au moins une encoche commençant au niveau de la première ouverture (6) de l'élément ressort (2) et au moins une deuxième encoche commençant au niveau de la deuxième ouverture (7).

3. Dispositif de serrage (1) selon la revendication 2, **caractérisé en ce que** l'encoche commençant au niveau de la première ouverture (6) est décalée dans la direction circonférentielle de l'élément ressort (2) par rapport à l'encoche commençant au niveau de la deuxième ouverture (7).

4. Dispositif de serrage (1) selon la revendication 2 ou 3, **caractérisé en ce que** les encoches à une extrémité opposée de l'ouverture (6, 7) de l'élément ressort (2) comprennent une cavité (9) supplémentaire.

5. Dispositif de serrage (1) selon l'une quelconque des revendications 1 à 4, **caractérisé en ce que** l'élément ressort (2) se compose d'un matériau résistant aux acides et à la corrosion.

6. Dispositif de serrage (1) selon l'une quelconque des revendications 1 à 5, **caractérisé en ce que** l'élément ressort (2) présente autour de la première et de la deuxième ouverture (6, 7) des surfaces d'actionnement (10) inclinées obliquement vers l'intérieur.

7. Instrument de préparation odontologique **caractérisé en ce que** l'instrument de préparation comprend un dispositif de serrage (1) selon l'une quelconque des revendications précédentes.
